(19) Europäisches Patentamt
European Patent Office
Office européen des brevets

(11) EP 3 225 229 A1

(12) # EUROPEAN PATENT APPLICATION

(43) Date of publication:
**04.10.2017 Bulletin 2017/40**

(21) Application number: **16162563.7**

(22) Date of filing: **29.03.2016**

(51) Int Cl.:
***A61K 8/33*** (2006.01) ***A61K 8/34*** (2006.01)
***A61K 8/36*** (2006.01) ***A61K 8/362*** (2006.01)
***A61K 8/365*** (2006.01) ***A61K 8/368*** (2006.01)
***A61Q 5/04*** (2006.01) ***A61Q 5/06*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Kao Germany GmbH**
**64297 Darmstadt (DE)**

(72) Inventors:
- **NÖCKER, Bernd**
**64297 DARMSTADT (DE)**
- **BREAKSPEAR, Steven**
**64297 DARMSTADT (DE)**
- **JIAN, Niu**
**64297 Darmstadt (DE)**

(74) Representative: **Grit, Mustafa**
**Kao Germany GmbH**
**Pfungstädter Strasse 98-100**
**64297 Darmstadt (DE)**

(54) **PROCESS FOR TREATING HAIR**

(57) The present invention relates to a process for treating hair wherein the hair is semi permanently shaped and subsequently reshaped with the application of several aqueous compositions.

EP 3 225 229 A1

## Description

The present invention relates to a process for treating hair wherein the hair is semi permanently shaped and subsequently reshaped with the application of several aqueous compositions.

Semi-permanent hair shaping has recently become widely used, especially for hair straightening with aqueous compositions comprising non reducing compounds. The hair straightened in this way holds its straightened shape for several hair washes to several months. However, this process does not allow consumers to have their hair subsequently adjusted into variable shapes and especially giving hair curls in order to increase the volume is not possible. In other words, these processes do not give any flexibility in subsequent hair shaping.

Well known traditional processes based on the use of reductive and oxidative compositions produces solid hair shapes which may not be changed without using additional chemical processes. Furthermore, these process do damage hair which results in loss of hair natural properties, especially strength and elasticity.

Resorcinol and its derivatives have been suggested for straightening hair in EP 2029236. The document focuses solely on straightening and does not provide any evidence of curling and flexible hair shaping.

The well-established glyoxylic acid based semi-permanent straighteners, such as in WO2012/010351, uses straightening iron and provides relatively long lasting straightening. Here again, there is no flexibility given of reshaping hair.

WO 2011/039098 discloses permanent styling process using two thiosiloxanes for providing long lasting styling benefits. According to the process, oxidative fixative is needed with the aid of hydrogen peroxide.

WO 2005/011626 discloses method of restyling hair using very hydrophobic, almost non-aqueous compositions. Hair is arranged in a defined shape and fixed physically with the composition. The effect is not long lasting.

The above summarized processes provide certain level of hair shaping which may be effective in the given shape but does not provide, or in some cases even make impossible, to change the hair style without using additional chemical process. The use of additional chemical processes on already chemically processed hair is harmful as it further damages hair which results in loss of hair natural properties.

The aim of the present invention is to find a new process for flexibly and durably shaping hair, especially curling and straightening semi permanently, and which makes change of hair shape possible without using any extensive chemical processes.

The present inventors have unexpectedly found that treating hair with a light reducing composition first and afterwards pretreating hair with an acidic composition comprising alpha hydroxyl acids and finally treating hair with a composition comprising one or more compounds selected from resorcinol and its derivatives and alpha hydroxyl acids makes possible obtaining durable larger curls and gives possibility to change hair style flexibly from straight to curly solely with the aid of hair styling tools such as irons, brushes, etc., and without using any additional chemical treatment process.

Accordingly, the first object of the present invention is a process for treating hair comprising the following steps:

a- Optionally cleansing hair,
b- Optionally drying the hair,
c- Applying hair a first aqueous composition comprising one or more reducing agent, one or more alkalizing agent and having a pH in the range of 7.1 to 11,
d- leaving the first composition on the hair for 1 to 30 min,
e- optionally rinsing off the first composition from the hair,
f- applying a second aqueous composition comprising one or more compounds of the general structure and/or a hydrate thereof and/or a salt thereof

R-CO-R'  Formula (I)

wherein R is selected from hydrogen, COOH, CN, optionally substituted $C_1$-$C_{10}$ alkyl, optionally substituted $C_2$-$C_{10}$ alkenyl, optionally substituted $C_2$-$C_{10}$ alkynyl, optionally substituted $C_3$-$C_{10}$ cycloalkyl, optionally substituted $C_6$-$C_{10}$ aryl or a 5-10-membered, optionally substituted heteroaryl group, wherein the optional substituents of the alkyl group are selected from halogen, hydroxyl, amino and $C_1$-$C_4$ alkoxy, and the optional substituents of the other groups are selected from halogen, hydroxyl, amino, $C_1$-$C_4$ alkyl and $C_1$-$C_4$ alkoxy and R' is COOH,
g- leaving the second composition on the hair for 1 to 2 min,
h- optionally rinsing off the composition from the hair,
i- applying a third aqueous composition onto hair comprising one or more compounds of the general structure of step f and one or more compounds according to general structure

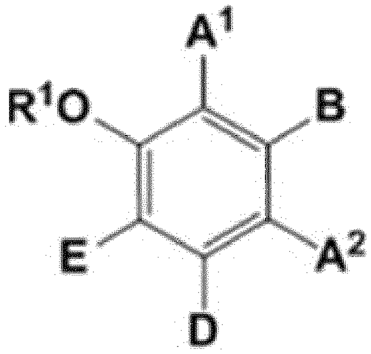

Formula II

wherein R1 is hydrogen or methyl, A1 and A2 are the same or different and are selected from hydrogen, C1 to C12 linear or branched alkyl or alkanyl, C7 to C12 aralkyl or alkenyl which may have a substituent, C1 to C6 linear or branched alkoxy or alkenyloxy, halogen or -COR2 wherein R2 is sleceted from C1 to C12 linear or branched alkyl or alkanyl, C7 to C12 aralkyl or alkenyl which may have a substituent or C6 to C12 aromatic hydrocarbon which may have a substituent, B is selected form hydrogen, C1 to C12 linear or branched alkyl or alkanyl, C7 to C12 aralkyl or alkenyl which may have a substituent, -OR3 or -COOR3 wherein R3 is selected from hydrogen, C1 to C6 linear or branched alkyl or alkanyl, D is selected from hydrogen, methyl or C1 to C12 linear or branched alkoxy or alkenyloxy, E is selected from hydrogen, methyl or C1 to C6 linear or branched alkoxy or alkenyloxy,and two or three of A1, A2, B and E are hydrogen and the others are not containing sulfonic group, and wherein A1 and B or A2 and B may be bonded mutually to form a benzene ring

j- heating the hair to a temperature in the range of 90 to 230°C,

k- leaving the third composition on the heated hair for 1 to 40 min,

l- optionally applying a fourth aqueous composition comprising one or more oxidizing agent and having an acidic pH,

m- optionally leaving the oxidizing composition on the hair for 1 to 10 min,

n- Optionally rinsing off the hair,

o- Optionally blow drying the hair.

Another object of the present invention is a kit for hair comprising the first, second and the third aqueous compositions as described above.

Still further object of the present invention is a kit for hair comprising

1- A first aqueous composition comprising one or more reducing agent, one or more alkalizing agent and having a pH in the range of 7.1 to 11,

2- A second aqueous composition comprising one or more compounds of the general structure given in step f above, and

3- Another aqueous composition comprising one or more compounds of the general structures given in step i above.

In case that a curly hair is given a curly shape, the hair is put on the curlers after the step h and prior to application of the third aqueous composition onto hair.

In the preferred embodiment of the present invention, the hair to be treated is dry and clean hair and therefore, prior to application of the aqueous reducing composition onto hair in step c, especially the dirty hair, is washed with a cleansing composition and dried. It should be noted that the treating humid, wet hair is not at all escluded from the scope of the invention.

The first aqueous composition comprises one or more reducing agents, one or more alkalizing agent and having a pH in the range of 7.1 to 11,

The reducing agents suitable are thiogylcolic acid and/or its salts, cysteamine and/or its salts, thioglycerin and/or its salts, glycerin esters of thioglycolic acid and/or its salts, thiolactic acid and/or its salts, cysteine or its derivatives and/or its salts and sodium sulfit. Preferred are thiogylcolic acid and/or its salts, thiolactic acid and/or its salts and cysteine or its derivatives and/or its salts. The most preferred is thiogylcolic acid and/or its salts.

One or more reducing agents are comprised in the first aqueous composition at a total concentration in the range of 0.1 % to 15%, preferably 0.2% to 12.5%, more preferably 0.5% to 10% and most preferably 0.5% to 5%, in particular 0.5 to 2% by weight, calculated to the total of the first composition.

The first aqueous composition comprises one or more alkalizing agents. Suitable ones are ammonia and alkyl- or alkanolamines according to the general structure

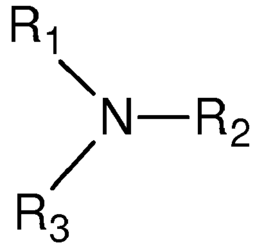

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents preferably selected from ammonia, monoethanolamine, and aminomethyl-propanol, and particularly suitable one is aminomethyl-propanol.

The alkalizing agent is comprised in the first aqueous composition at a total concentration of 0.25% to 10%, preferably 0.5% to 7.5%, more preferably 0.75% to 5% and most preferably 1 % to 4% by weight calculated to the total of the first aqueous composition.

The first aqueous composition has a pH in the range of 7.1 to 11, preferably 8 to 11, and more preferably 8 to 10.5 and most preferably 8.5 to 10 measured at 20°C.

In addition to the above mentioned alkalizing agents, alkali carbonates are preferably comprised in the compositions which may also be seen as additional alkalizing/buffering agent. Suitable ones are carbonate and bicarbonate alkali salts such as sodium, potassium and ammonium salts. The preferred are bicarbonate salts and especially preferred is ammonium bicarbonate.

The carbonates are comprised at a total concentration in the range of 0.25% to 10%, preferably 0.5% to 7.5%, more preferably 0.75% to 5% and most preferably 1% to 4% by weight calculated to the total of the first aqueous composition.

After application of the first aqueous composition onto hair, it is left on the hair for 1 to 30 min at ambient temperature, preferably 2 to 20 min and more preferably 5 to 15 min.

The aqueous first composition is preferably rinsed off from hair at the end of the processing time.

Subsequently hair is applied a second aqueous composition comprising one or more compounds of the general structure and/or a hydrate thereof and/or a salt thereof

R-CO-R' Formula (I)

wherein R is selected from hydrogen, COOH, CN, optionally substituted $C_1$-$C_{10}$ alkyl, optionally substituted $C_2$-$C_{10}$ alkenyl, optionally substituted $C_2$-$C_{10}$ alkynyl, optionally substituted $C_3$-$C_{10}$ cycloalkyl, optionally substituted $C_6$-$C_{10}$ aryl or a 5-10-membered, optionally substituted heteroaryl group, wherein the optional substituents of the alkyl group are selected from halogen, hydroxyl, amino and $C_1$-$C_4$ alkoxy, and the optional substituents of the other groups are selected from halogen, hydroxyl, amino, $C_1$-$C_4$ alkyl and $C_1$-$C_4$ alkoxy and R' is COOH.

The preferred compounds of Formula (I) are glyoxylic acid, pyruvic acid and 2-ketobutyric acid.

The compounds of Formula (I) may be comprised in the composition in its free acid form. The carbonyl group adjacent to the carboxyl group of the acid may also be present in the hydrate form. Apart from the free acid form and the hydrate thereof, salts of the acid or the hydrate may also be used.

The hydrate of the acid of Formula (I) may be formed when providing the composition as an aqueous solution. For instance, glyoxylic acid (H-CO-COOH) in aqueous solution is almost quantitatively present as the hydrate (H-$C(OH)_2$-COOH). Besides, the hydrate may also condense to dimers.

In the present invention, glyoxylic acid is the most preferred carboxylic acid of Formula (I).

The total concentration of one or more compounds of the formula (I) and/or a hydrate thereof and/or salts thereof is in the range of 0.1 % to 20%, preferably 0.5% to 15%, more preferably 0.75% to 12.5% and even more preferably 1 % to 10% by weight, calculated to the total of the second aqueous composition.

The pH of the second aqueous composition is in the range of 1 to 4.0, preferably in the range of 1.5 to 4, more preferably 2 to 3, as measured directly and at ambient temperature. The pH of the compositions may be adjusted using known alkaline solutions, preferably with sodium hydroxide solution.

The second aqueous composition is left on the hair for 1 to 20 min, preferably 2 to 15 min and more preferably 2.5 to 10 min at ambient temperature.

Subsequently and without rinsing off the second aqueous composition, the hair is put on curlers having various diameters in case curly hair is the targeted shape.

In a further step, the hair is applied the third aqueous composition comprising one or more compounds of the Formula (I) above and one or more compounds of Formula (II).

The third aqueous composition comprises one or more compounds of the Formula (1) at a total concentration in the range of 0.1% to 20%, preferably 0.5% to 15%, more preferably 0.75% to 12.5% and even more preferably 1% to 10% by weight, calculated to the total of the second aqueous composition.

The third aqueous composition comprises one or more compounds of Formula (II) at a total concentration in the range of 0.1 % to 15%, preferably 0.25% to 12.5%, more preferably 0.5% to 10% and even more preferably 1% to 7.5% by weight, calculated to the total of the second aqueous composition

In a preferred embodiment of the present invention, the third aqueous composition comprises the one or more compounds of the Formula (1) and Formula(II) at a weight ratio in the range of 1:0.1 to 1:1, preferably 1:0.2 to 1:0.8 and more preferably 1:0.25 to 1:0.7 and most preferably 1:0.35 to 1:0.6.

In a further preferred embodiment of the present invention is that in order to secure the optimal stability and consequently the optimal effects of the process, the third aqueous composition is prepared immediately prior to application onto hair in one of the following ways

i- mixing the two aqueous compositions comprising the compounds of Formula (1) and compounds of Formula (II),
ii- adding solid compounds of Formula (II) to the aqueous composition comprising the compounds of Formula (1),
iii- adding the solid compounds of Formula (1) to the aqueous composition comprising one or more compounds of Formula (II).

The pH of the third aqueous composition is in the range of 1.5 to 5, preferably 2.5 to 4.5, more preferably 2.5 to 4.

The suitable compounds according to Formula (II) are resorcinol, 2-methyl resorcinol, 4-n-butyl resorcinol, 4-phenyl ethyl resorcinol, 4-chlorresorcinol, resveratrol, phloretin and 2,2',4,4'-tetrahydroxybenzophenone. The preferred are resorcinol, 2-methyl resorcinol, 4-phenyl ethyl resorcinol and resveratrol. The more preferred are resorcinol and 4-phenyl ethyl resorcinol. The most preferred is resorcinol.

The third aqueous composition is left on the hair for a period of 1 to 60 min, preferably 2 to 45 min, more preferably 5 to 40 min and most preferably 5 to 30 min at a temperature in the range of 90 to 230°C, preferably in the range of 100 to 200°C, more preferably 110 to 180°C and most preferably 120 to 180°C. Heating up the hair may be carried out with various tools such as irons, digital perming machine, in case curly hair is the target, which heats up the curlers and the heat is transferred to the hair. It has been observed that optimal and safe operation of the digital curling rods is achieved at a temperature 140°C +/- 10°C.

Since the third aqueous composition may be left on the hair for considerably long period of time at temperatures far higher than 100°C, it is beneficial to cover the hair with a device which helps to keep the humidity within the close vicinity of hair, i.e. prevents evaporation of water and consequently drying the hair.

The hair treated with the third aqueous composition with heat is subsequently, optionally treated with an aqueous oxidizing composition comprising one or more oxidizing agents and having an acidic pH.

Suitable oxidizing agents are hydrogen peroxide, urea peroxide, melamine peroxide or perborate salts. The most preferred is hydrogen peroxide. The composition comprises one or more oxidizing agents at a total concentration of 0.1 % to 5% by weight, preferably 0.25% to 3%, more preferably 0.5% to 2.5% and most preferably 0.5% to 2% by weight, calculated to total of the third composition. The pH of the composition is in the range of 1.5 to 5, preferably 2.5 to 4.5, more preferably 2.5 to 4.

Although it may be possible to leave in the compositions applied onto hair and finishing the process without rinsing off the hair with water, rinsing off after finishing the obligatory treatment steps is preferred in order to maintain the natural cosmetic properties of hair, especially uncoated/unloaded feel and soft feeling upon touching.

In the aqueous second and third compositions, especially aqueous third composition, the aromatic compounds are not comprised having at least one amino group and another amine or hydroxyl group at o or p position to the amine group wherein the amine groups may additionally be substituted and in case they are present in the composition their concentration must be less than 1% in order to prevent any oxidative color development under the used conditions, preferably the third and the second composition does not comprise any reducing agent.

In a further embodiment of the present invention, any of the compositions may comprise one or more thickening polymers selected from anionic, nonionic, cationic and amphoteric polymers, preferably selected from polymers with a viscosity of at least 500 mPa·s measured at a polymer concentration of 1% by weight in water and at 20°C with a Brookfield viscometer, such as at 10 rpm for 1 min, with an appropriate spindle.

Suitable polymers are cellulose polymers, alginates, polysaccarides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are hydroxyethylcellulose, dehydroxanthan gum, xanthan gum, guar gum and polymeric anionic thickeners Carbomer and its derivatives. The particularly preferred thickening agent is dehydroxanthan gum. The thickening agents are preferably comprised in the compositions at a total concentration in the range of 0.1 % to 2%, preferably, 0.2% to 1.75%, more preferably 0.25% to 1.5% and most preferably 0.3% to 1.25% by weight, calculated to the total of each aqueous composition. It should be noted that thickening is necessary in order to keep the aqueous compositions on the hair during their processing time. On the other hand, the selected consistency of the compositions and, especially the third aqueous composition, should be selected carefully in order not to prevent penetration of the composition into the hair bundles put on curlers.

Any of the compositions, first, second and third aqueous compositions may comprise one or more of the commonly used hair conditioning compounds. These compounds are for example fatty alcohols, surfactants such as anionic, nonionic, cationic and amphoteric ones, ubiquinones, ceramides, organic solvents, lipophilic ingredients such as vegetable oils, mineral oils, silicones, fatty acid fatty alcohol esters, preservatives, amino acids, and polyols. It should be noted that these compounds are optionally comprised in the any of the compositions and their incompatibility must be carefully considered prior to addition in the compositions.

Any of the compositions may comprise one or more fatty alcohols. In particular the compositions may further be in the form of an emulsion and then comprises preferably one or more fatty alcohols and one or more surfactants as emulsifier. Suitable fatty alcohols are the ones with the chain length of 14 to 22 C atoms which may be saturated or unsaturated, linear or branched which may as well be substituted. Non-limiting examples are myristyl alcohol, cetyl alcohol, stearyl alcohol, behenyl alcohol and cetostearyl alcohol. The total concentration of fatty alcohol is in the range from 0.1 % to 7.5%, preferably 0.2% to 5% by weight, calculated to the total of each composition.

The surfactants suitable are selected from anionic, nonionic, amphoteric and/or cationic surfactants. The anionic, nonionic, amphoteric surfactants are used generally as emulsifier or solubilizer whereas the cationic surfactants are at the same time particularly used as hair conditioners.

Anionic surfactants suitable are in principle known from the cleansing compositions. These are anionic surfactants of the sulfate, sulfonate, carboxylate and alkyl phosphate type, for example, the known $C_{10}$-$C_{18}$-alkyl sulfates, and in particular the respective ether sulfates, for example, $C_{12}$-$C_{14}$-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are $\alpha$-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO®" and "AKYPO-SOFTR®".

Further suitable anionic surfactants are also $C_8$-$C_{22}$-acyl aminocarboxylic acids or the water-soluble salts thereof. Especially preferred is N-lauroyl glutamate, in particular as sodium salt, as well as, for example, N-lauroyl sarcosinate, N-$C_{12}$-$C_{18}$-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

Further suitable surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics$^R$", as well as fatty alcohol ethoxylates, $C_{10}$-$C_{22}$-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also been proven suitable.

Suitable cationic surfactants are according to the general structure

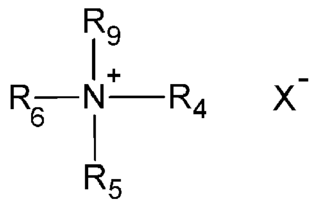

where $R_5$ is a saturated or unsaturated, branched or linear alkyl chain with 8-22 C atoms or

$R_7$ CO NH $(CH_2)_n$

where $R_7$ is saturated or unsaturated, branched or linear alkyl chain with 7-21 C atoms and n has typical value of 1 - 4 or

$R_8$ CO O $(CH_2)_n$

where $R_8$ is saturated or unsaturated, branched or linear alkyl chain with 7-21 C atoms and n has typical value of 1 - 4, and

$R_4$ is H or unsaturated or saturated, branched or linear alkyl chain with 1 - 22 C atoms or

$R_7$ CO NH $(CH_2)_n$

or

$R_8$ CO O $(CH_2)_n$

where $R_7$, $R_8$ and n are same as above.

$R_9$ and $R_6$ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The concentration of total surfactants in any of the compositions is in the range of 0.1% to 10%, preferably 0.2% to 7.5% and most preferably 0.2% to 5% by weight, calculated to the total of each composition.

The aqueous compositions may further comprise lipophilic ingredients such as vegetable oils, for example, jojoba oil or any other; liquid paraffins, especially paraffinum perliquidum and parafiinum subliquidum; silicones for example linear polysiloxanes such as dimethicones with various consistency and dimethiconols, aminated silicones with secondary, tertiary or quaternary ammonium groups such as polysilicone 9, and quaternium 80, cyclic silicones such as cyclomethicones, arylated silicones such as phenyl trimethicone; fatty acid esters such as octyl palmitate, isocetyl palmitate, isopropyl palmitate and octyl stearate, $C_{10}$- to $C_{36}$-fatty acid triglycerides, as well as their mixtures. Total concentration of these lipophilic compounds is in the range of 0.1 % to 20% by weight, preferably from 1 % to 15% by weight, and more preferably from 2% to 10% by weight, calculated to the total of each composition.

The compositions may also comprise cationic polymers as conditioning and/or thickening agents. Those are cationic cellulose type polymers know as Polymer JR type from Amerchol such as Polyquaternium 10 or cationic guar gum known with trade name Jaguar from Rhone-Poulenc and chemically for example Guar hydroxypropyl trimonium chloride. Furthermore, chitosan and chitin can also be included in the compositions as cationic natural polymers.

Furthermore, it has been found suitable to use those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

The total concentration of cationic polymers may be in the range of 0.1 % to 7.5% by weight, preferably 0.3% to 5% by weight and more preferably 0.5% to 2.5% by weight, calculated to the total of each composition

The compositions may comprise one or more ceramide compound, such as the one according to general formula

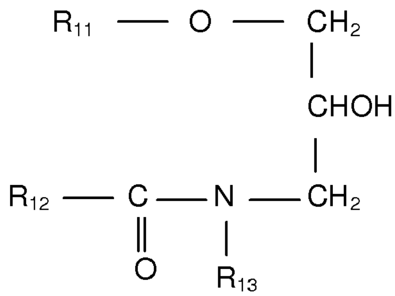

where $R_{11}$ and $R_{12}$ are independent from each other alkyl- or. alkenyl group with 10 to 22 carbon atoms, $R_{13}$ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01 % to 2%, preferably 0.01 % to 1 % by weight calculated to the total or each composition.

The compositions may comprise ubiquinone of the formula:

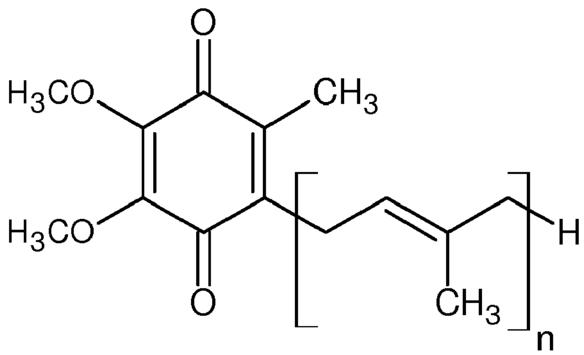

wherein n is a number from 1 to 10. The concentration of ubiquinone can vary between 0.001% and 10% by weight, calculated to the total of each composition.

The compositions may comprise one or more organic solvent such as 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol. Concentration of one or more organic solvent is in the range of 0.1% to 15%, preferably 0.5% to 12.5% and more preferably 1 % to 10% and most preferably 1% to 7.5% by weight calculated to the total of each composition.

The compositions A, B, C, and/or D may further comprise one or more polyols, preferably at a concentration in the range of 0.01 % to 5%, preferably 0.1 % to 3% and more preferably 0.2% to 2.5% and most preferably 0.25% to 2% by weight calculated to the total of each composition. Suitable ones are propylene glycol, diproplylene glycol, glycerine, panthenol and its derivatives.

The compositions may further comprise any known preservatives if necessary, fragrance and any other cosmetically acceptable ingredient.

The following examples are to illustrate the invention but not to limit.

## Example 1

The following compositions were prepared and used in the tests in order to show comparatively and objectively the effect of the invention.

| Compositions (all values are % by weight) | | | | | | | |
|---|---|---|---|---|---|---|---|
| Process step | Component | 1 | 2 | 3 | 4 | 5 | 6 |
| | | | | | | | |
| A | Ammonium thioglycolate | 2,00 | 2,00 | 2,00 | - | - | - |
| | Ammonium hydroxide | q.s. to pH 10 | | | - | - | - |

(continued)

| Compositions (all values are % by weight) | | | | | | | |
|---|---|---|---|---|---|---|---|
| Process step | Component | 1 | 2 | 3 | 4 | 5 | 6 |
| | Water | to 100 | | | - | - | - |
| | | | | | | | |
| B | Glyoxylic acid | 5,00 | 5,00 | - | 5,00 | | |
| | Sodium hydroxide | q.s. to pH 2.0 | | - | q.s. to pH 2.0 | | |
| | Water | to 100 | | - | | | |
| | | | | | | | |
| C | Glyoxylic acid | 10,00 | 10,00 | 10,00 | 10,00 | 15,00 | - |
| | Resorcinol | 5,00 | - | 5,00 | 5,00 | - | |
| | 2-Methyl resorcinol | - | 5,00 | - | - | - | 5,00 |
| | Sodium hydroxide | q.s. to pH 3.5 | | | | | |
| | Water | to 100 | | | | | |
| Evaluation | Curl ratio (%) | 48 | 46 | 35 | 31 | 6 | 4 |

The above compositions were prepared and used for treating hair. Therefore, hair streaks with a length of 20 cm were used. The first composition, if required according to the table, was applied onto shampooed and dried hair in a hair to composition weight ratio of 1:1 and left on the hair for 5 min and rinsed off from hair. Afterwards the composition B, if required according to the table, was applied onto hair at the same weight ratio and left on the hair for additional 5 min and without rinsing off from hair, the hair was put on curlers of the digital perm machine and the rods were heated to 140°C (measured on the hair with an infrared digital thermometer from a distance of 15 cm) and the hair was applied the third composition, composition C and let on the hair for 20 min. The hair was covered with an aluminum sheet during processing in the third step. Afterwards the hair was taken off from the digital perming machine and the length was measured and the curl ratio was calculated with the following equation. In case the hair was treated with only one or two composition, the other steps were simply not applied.

$$\text{Curl ratio (CR)} = ((L0 - Lt)/L0) \times 100$$

Wherein L0 is the length of the hair prior to the treatment, Lt is the lenth of the hair after the treatment.

From the above results it is clear that when hair is treated with all three compositions the highest curl ratio (CR) was obtained. In case the hair was not treated with the reducing composition the curl ratio was considerably reduced. In case the two steps were completely absent, the curl ratio was very low, almost negligible.

Additionally, the tress treated with the example 1 - 1, was straightened using a flat iron having a surface temperature of 230°C. The hair was treated 5 times with 3 sec heat application period and it was observed that the curly hair was straightened.

The same straightened hair was then again curled with curling iron operated at 180°C for 30 sec and repeated 3 times the same and it was observed that the hair was curled again and had the similar curl retention value.

Similar results are observed with the following examples using the same method.

## Example 2

| Process step | Component | % by weight |
|---|---|---|
| | | |
| A | Ammonium thioglycolate | 2 |
| | Ammonium hydrogen carbonate | 2 |

(continued)

| Process step | Component | % by weight |
|---|---|---|
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | Resorcinol | 6 |
| | Sodium hydroxide | q.s. to pH 3.5 |
| | Water | q.s. to 100 |

**Example 3**

| Process step | Component | % by weight |
|---|---|---|
| | | |
| A | Ammonium thioglycolate | 2 |
| | Ammonium hydrogen carbonate | 2 |
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | 2-methyl resorcinol | 6 |
| | Sodium hydroxide | q.s. to pH 3.5 |
| | Water | q.s. to 100 |

**Example 4**

| Process step | Component | % by weight |
|---|---|---|
| | | |
| A | Ammonium thioglycolate | 2 |
| | Ammonium hydrogen carbonate | 2 |

(continued)

| Process step | Component | % by weight |
|---|---|---|
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | 2-methyl resorcinol | 6 |
| | Sodium hydroxide | q.s. to pH 3.5 |
| | Water | q.s. to 100 |

**Example 5**

| Process step | Component | % by weight |
|---|---|---|
| | | |
| A | Ammonium thioglycolate | 2 |
| | Ammonium hydrogen carbonate | 2 |
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | 4-n-butyl resorcinol | 6 |
| | Sodium hydroxide | q.s. to pH 3.5 |
| | Water | q.s. to 100 |

**Example 6**

| Process step | Component | % by weight |
|---|---|---|
| | | |
| A | Ammonium thioglycolate | 2 |
| | Ammonium hydrogen carbonate | 2 |

(continued)

| Process step | Component | % by weight |
|---|---|---|
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | 4-phenyl ethyl resorcinol | 6 |
| | Sodium hydroxide | q.s. to pH 3.5 |
| | Water | q.s. to 100 |

**Example 7**

| Process step | Component | % by weight |
|---|---|---|
| | | |
| A | Ammonium thioglycolate | 2 |
| | Ammonium hydrogen carbonate | 2 |
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | 4-chlor resorcinol | 6 |
| | Sodium hydroxide | q.s. to pH 3.5 |
| | Water | q.s. to 100 |

**Example 8**

| Process step | Component | % by weight |
|---|---|---|
| | | |
| A | Ammonium thioglycolate | 2 |
| | Ammonium hydrogen carbonate | 2 |

(continued)

| Process step | Component | % by weight |
|---|---|---|
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | 2,2',4,4'-tetrahydroxybenzophenone | 6 |
| | Sodium hydroxide | q.s. to pH 3.5 |
| | Water | q.s. to 100 |

**Example 9**

| Process step | Component | % by weight |
|---|---|---|
| | | |
| A | Ammonium thioglycolate | 2 |
| | Ammonium hydrogen carbonate | 2 |
| | Ammonium hydroxide | q.s. to pH 10 |
| | Water | q.s. to 100 |
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | Resveratrol | 6 |
| | Sodium hydroxide | q.s. to pH 3.5 |
| | Water | q.s. to 100 |

**Example 10**

| Process step | Component | % by weight |
|---|---|---|
| | | |
| A | Ammonium thioglycolate | 2 |
| | Ammonium hydrogen carbonate | 2 |
| | Ammonium hydroxide | q.s. to pH 10 |
| | Water | q.s. to 100 |

(continued)

| Process step | Component | % by weight |
|---|---|---|
| | | |
| B | Glyoxylic acid | 5 |
| | Sodium hydroxide | q.s. to pH 2.0 |
| | Water | q.s. to 100 |
| | | |
| C | Glyoxylic acid | 12 |
| | Phloretin | 6 |
| | Sodium hydroxide | q.s. to pH 3.5 |
| | Water | q.s. to 100 |

## Claims

**1.** A process for treating hair comprising the following steps:

a- Optionally cleansing hair,
b- Optionally drying the hair,
c- Applying hair a first aqueous composition comprising one or more reducing agent, one or more alkalizing agent and having a pH in the range of 7.1 to 11,
d- leaving the first composition on the hair for 1 to 30 min,
e- optionally rinsing off the first composition from the hair,
f- applying a second aqueous composition comprising one or more compounds of the general structure and/or a hydrate thereof and/or a salt thereof

R-CO-R' Formula (I)

wherein R is selected from hydrogen, COOH, CN, optionally substituted $C_1$-$C_{10}$ alkyl, optionally substituted $C_2$-$C_{10}$ alkenyl, optionally substituted $C_2$-$C_{10}$ alkynyl, optionally substituted $C_3$-$C_{10}$ cycloalkyl, optionally substituted $C_6$-$C_{10}$ aryl or a 5-10-membered, optionally substituted heteroaryl group, wherein the optional substituents of the alkyl group are selected from halogen, hydroxyl, amino and $C_1$-$C_4$ alkoxy, and the optional substituents of the other groups are selected from halogen, hydroxyl, amino, $C_1$-$C_4$ alkyl and $C_1$-$C_4$ alkoxy and R' is COOH,
g- leaving the second composition on the hair for 1 to 2 min,
h- optionally rinsing off the composition from the hair,
i- applying a third aqueous composition onto hair comprising one or more compounds of the general structure of step f and one or more compounds according to general structure

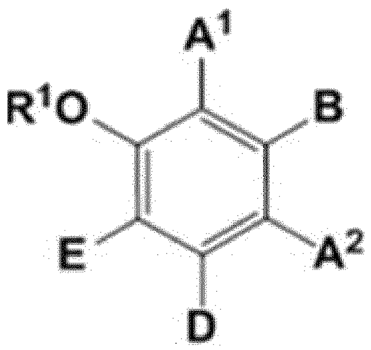

Formula II

wherein R1 is hydrogen or methyl, A1 and A2 are the same or different and are selected from hydrogen, C1 to

C12 linear or branched alkyl or alkanyl, C7 to C12 aralkyl or alkenyl which may have a substituent, C1 to C6 linear or branched alkoxy or alkenyloxy, halogen or -COR2 wherein R2 is sleceted from C1 to C12 linear or branched alkyl or alkanyl, C7 to C12 aralkyl or alkenyl which may have a substituent or C6 to C12 aromatic hydrocarbon which may have a substituent, B is selected form hydrogen, C1 to C12 linear or branched alkyl or alkanyl, C7 to C12 aralkyl or alkenyl which may have a substituent, -OR3 or -COOR3 wherein R3 is selected from hydrogen, C1 to C6 linear or branched alkyl or alkanyl, D is selected from hydrogen, methyl or C1 to C12 linear or branched alkoxy or alkenyloxy, E is selected from hydrogen, methyl or C1 to C6 linear or branched alkoxy or alkenyloxy,and two or three of A1, A2, B and E are hydrogen and the others are not containing sulfonic group, and wherein A1 and B or A2 and B may be bonded mutually to form a benzene ring
j- heating the hair to a temperature in the range of 90 to 230°C,
k- leaving the third composition on the heated hair for 1 to 40 min,
l- optionally applying a fourth aqueous composition comprising one or more oxidizing agent and having an acidic pH,
m- optionally leaving the oxidizing composition on the hair for 1 to 10 min,
n- Optionally rinsing off the hair,
o- Optionally blow drying the hair.

**2.** The process according to claim 1 wherein the hair is put on the curlers after the step g or h and prior to application of the third aqueous composition onto hair in step i.

**3.** The process according to claims 1 and/or 2 wherein the reducing agent is selected from thiogylcolic acid and/or its salts, cysteamine and/or its salts, thioglycerin and/or its salts, glycerin esters of thioglycolic acid and/or its salts, thiolactic acid and/or its salts, cysteine or its derivatives and/or its salts and sodium sulfit, preferably it is thiogylcolic acid and/or its salts, and preferably present at a total concentration in the range of 0.1 to 15% by weight, calculated to the total of the first composition.

**4.** The process according to any of the preceding claims wherein the alkalizing agent is selected from ammonia and alkyl- or alkanolamines according to the general structure

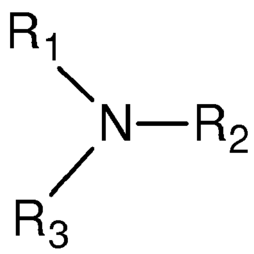

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents are preferably selected from ammonia, monoethanolamine, and aminomethyl-propanol, and particularly suitable one is aminomethyl-propanol and preferably present at a total concentration in the range of 0.25 to 10% by weight, calculated to the total of the first composition.

**5.** The process according to any of the preceding claims wherein the first composition comprises an alkali carbonate and/or an alkali bicarbonate, preferably selected from carbonate and bicarbonate alkali salts such as sodium, potassium and ammonium salts, preferably it is ammonium bicarbonate, preferably comprised at a total concentration in the range of 0.25 to 10% by weight, calculated to the total of the first composition.

**6.** The process according to any of the preceding claims wherein the second aqueous composition comprises glyoxylic acid and/or its salts and/or its dimers, preferably at a total concentration in the range of 0.1 to 20%, by weight, calculated to the total of the first composition.

**7.** The process according to any of the preceding claims wherein the third aqueous composition comprises glyoxylic acid and/or its salts and/or its dimers, preferably at a total concentration in the range of 0.1 to 20%, by weight, calculated to the total of the first composition, as the compound according to the Formula (I), preferably at a total concentration in the range of 0.1 to 20%, by weight, calculated to the total of the first composition.

**8.** The process according to any of the preceding claims wherein the third aqueous composition comprises one or more compounds of Formula (II) at a total concentration in the range of 0.1 to 15%, by weight, calculated to the total of the first composition.

**9.** The process according to any of the preceding claims wherein the third aqueous composition comprises one or more compounds according to the Formula (II) selected from resorcinol, 2-methyl resorcinol, 4-n-butyl resorcinol, 4-phenyl ethyl resorcinol, 4-chlorresorcinol, resveratrol, phloretin and 2,2',4,4'-tetrahydroxybenzophenone.

**10.** The process according to any of the preceding claims wherein the compound according to the Formula (II) in the third aqueous composition is resorcinol and/or 2-methyl resorcinol.

**11.** The process according to any of the preceding claims wherein the third aqueous composition has a pH in the range of 1.5 to 5.

**12.** The process according to any of the preceding claims wherein the second and third compositions, especially the third composition, does not comprise compounds having at least one amino group and another amine or hydroxyl group at o or p position to the amine group wherein the amine groups may additionally be substituted and in case they are present in the composition, their concentration must be less than 1% in order to prevent any oxidative color development under the used conditions, and preferably the third and the second composition does not comprise any reducing agent.

**13.** The process according to any of the preceding claims wherein any of the first, second and third compositions comprise one or more thickening polymers selected from anionic, nonionic, cationic and amphoteric polymers, preferably selected from polymers with a viscosity of at least 500 mPa·s measured at a polymer concentration of 1 % by weight in water and at 20°C with a Brookfield viscometer, such as at 10 rpm for 1 min, with an appropriate spindle, preferably selected from cellulose polymers, alginates, polysaccarides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives, preferably comprised in the compositions at a total concentration in the range of 0.1 % to 2% by weight, calculated to the total of each aqueous composition.

**14.** The process according to any of the preceding claims wherein any of the first, second and third compositions comprise one or more hair conditioning compounds, preferably selected from fatty alcohols, surfactants such as anionic, nonionic, cationic and amphoteric ones, ubiquinones, ceramides, organic solvents, lipophilic ingredients such as vegetable oils, mineral oils, silicones, fatty acid fatty alcohol esters, preservatives, amino acids, and polyols.

**15.** Kit for hair comprising

1- a first aqueous composition comprising one or more reducing agent, one or more alkalizing agent and having a pH in the range of 7.1 to 11,
2- a second aqueous composition comprising one or more compounds of the general structure given in step f above, and
3- another aqueous composition comprising one or more compounds of the general structures given in step i above.

Europäisches Patentamt
European Patent Office
Office européen des brevets

# EUROPEAN SEARCH REPORT

Application Number
EP 16 16 2563

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/067702 A1 (KAO CORP [JP]) 8 May 2014 (2014-05-08) * page 1, line 6 - line 7 * * page 5, line 11ff * * page 9, line 9ff * * page 11, line 25ff * * claims; examples * ----- | 1-15 | INV. A61K8/33 A61K8/34 A61K8/36 A61K8/362 A61K8/365 A61K8/368 A61Q5/04 A61Q5/06 |
| Y | WO 2014/068102 A2 (KAO CORP [JP]) 8 May 2014 (2014-05-08) * page 1, line 5 - line 6; claims; examples * * page 5, line 10ff * * page 13, paragraph 12ff * * page 15, line 5ff * ----- | 1-15 | |
| Y | WO 2014/068101 A2 (KAO CORP [JP]) 8 May 2014 (2014-05-08) * page 1, line 4 - line 5 * * page 4, line 27ff * * page 6, line 15ff * * page 9, line 4 - line 17 * * page 11, line 1ff; claims; examples * ----- | 1-15 | TECHNICAL FIELDS SEARCHED (IPC) |
| Y | DE 20 2013 011675 U1 (OREAL [FR]) 12 February 2014 (2014-02-12) * paragraph [0014] * * paragraph [0025] - paragraph [0031]; claims; examples * ----- | 1-15 | A61K A61Q |
| Y | WO 2011/104282 A2 (ALDERAN S A S DI D OTTAVI ADELE & C [IT]; MANNOZZI ALDERANO [IT]) 1 September 2011 (2011-09-01) * page 1, line 1 - line 2 * * page 3, line 16 - line 23 * * claims; examples * ----- -/-- | 1-15 | |

The present search report has been drawn up for all claims

1

| Place of search | Date of completion of the search | Examiner |
|---|---|---|
| Munich | 12 May 2016 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 16 2563

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DE 10 2009 001483 A1 (HENKEL AG & CO KGAA [DE]) 28 January 2010 (2010-01-28)<br>* paragraphs [0001], [0046], [0111]; claims; examples *<br>----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 12 May 2016 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 16 2563

This annex lists the patent family members relating to the patent documents cited in the above-mentioned European search report. The members are as contained in the European Patent Office EDP file on
The European Patent Office is in no way liable for these particulars which are merely given for the purpose of information.

12-05-2016

| Patent document cited in search report | Publication date | Patent family member(s) | Publication date |
|---|---|---|---|
| WO 2014067702 A1 | 08-05-2014 | AU 2013339733 A1 | 21-05-2015 |
| | | CN 104768617 A | 08-07-2015 |
| | | EP 2914346 A1 | 09-09-2015 |
| | | JP 2015535268 A | 10-12-2015 |
| | | US 2015290096 A1 | 15-10-2015 |
| | | WO 2014067702 A1 | 08-05-2014 |
| WO 2014068102 A2 | 08-05-2014 | DE 202013011967 U1 | 09-02-2015 |
| | | WO 2014068102 A2 | 08-05-2014 |
| WO 2014068101 A2 | 08-05-2014 | NONE | |
| DE 202013011675 U1 | 12-02-2014 | DE 202013011675 U1 | 12-02-2014 |
| | | EP 2916804 A1 | 16-09-2015 |
| | | FR 2997846 A1 | 16-05-2014 |
| | | US 2015313816 A1 | 05-11-2015 |
| | | WO 2014072490 A1 | 15-05-2014 |
| WO 2011104282 A2 | 01-09-2011 | AU 2011219795 A1 | 18-10-2012 |
| | | BR PI1002742 A2 | 29-05-2012 |
| | | CN 102970962 A | 13-03-2013 |
| | | EP 2538916 A2 | 02-01-2013 |
| | | IT 1398503 B1 | 01-03-2013 |
| | | JP 2013520468 A | 06-06-2013 |
| | | JP 2016047851 A | 07-04-2016 |
| | | RU 2012140303 A | 27-03-2014 |
| | | SG 183477 A1 | 27-09-2012 |
| | | SG 10201406083S A | 27-11-2014 |
| | | US 2012312317 A1 | 13-12-2012 |
| | | WO 2011104282 A2 | 01-09-2011 |
| DE 102009001483 A1 | 28-01-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2029236 A
- WO 2012010351 A
- WO 2011039098 A
- WO 2005011626 A